(19) Europäisches Patentamt
European Patent Office
Office européen des brevets

(11) **EP 1 990 050 A1**

(12) **EUROPEAN PATENT APPLICATION**
published in accordance with Art. 158(3) EPC

(43) Date of publication:
**12.11.2008 Bulletin 2008/46**

(21) Application number: **07714132.3**

(22) Date of filing: **14.02.2007**

(51) Int Cl.:
*A61K 31/155* (2006.01)   *A61K 9/06* (2006.01)
*A61K 47/10* (2006.01)   *A61K 47/12* (2006.01)
*A61K 47/14* (2006.01)   *A61K 47/16* (2006.01)

(86) International application number:
**PCT/JP2007/052554**

(87) International publication number:
**WO 2007/094332 (23.08.2007 Gazette 2007/34)**

(84) Designated Contracting States:
**AT BE BG CH CY CZ DE DK EE ES FI FR GB GR HU IE IS IT LI LT LU LV MC NL PL PT RO SE SI SK TR**

(30) Priority: **15.02.2006 JP 2006038652**

(71) Applicant: **Maruishi Pharmaceutical Co., Ltd.**
**Osaka-shi,**
**Osaka 541-0044 (JP)**

(72) Inventors:
• **IKEDA, Masahiro**
**Osaka-shi, Osaka 538-0042 (JP)**
• **NISHIHARA, Yutaka**
**Osaka-shi, Osaka 538-0042 (JP)**

(74) Representative: **Scheuermann, Erik et al**
**Witte, Weller & Partner**
**Patentanwälte**
**Postfach 10 54 62**
**70047 Stuttgart (DE)**

(54) **DISINFECTANT OINTMENT**

(57)    Disclosed is a microbicidal and antiseptic ointment which contains at least one kind selected from the group consisting of chlorhexidine and its salt in an amount from 0.1 to 4 mass % and a hydrocarbon alcohol having 2 to 3 carbon atoms in an amount from 8 to 25 mass %, and sustains a microbicidal effect for at least 6 hours that is approved by a test method for evaluation of hand antisepsis.

Fig. 5

microbial reduction effect (in vivo palm stamp method)

residual ratio of microbes (%)

— preparation 1
— preparation 2
— reference preparation 1
— reference preparation 3
— ethanol for disinfection

time (hr)

**Description**

TECHNICAL FIELD

The present invention relates to a microbicidal and antiseptic ointment for application on human hands or skin to provide both fast-acting and persistent antiseptic effect.

BACKGROUND ART

Chlorhexidine gluconate, which was developed in U.K. in 1954, is a strong basic substance with a strong antimicrobial activity. The antimicrobial activity (microbicidal and antiseptic activity) is considered to result from the following mechanisms. Chlorhexidine gluconate disrupts a microbial cytoplasmic membrane at a relatively low concentration, thereby causing irreversible leakage of its intracellular components and enzymatic inhibition, while it causes intracellular precipitation of proteins and nucleic acids at a relatively higher concentration. Chlorhexidine gluconate is, today, used for a diversity of disinfection purposes, such as disinfection of hands or skin, surgical sites, medical apparatus, and the like. Chlorhexidine gluconate is used in the form of an aqueous or ethanol solution, or the like, depending on diverse purposes (see also, Manual of the Japanese Pharmacopoeia, 14th Edition, 2001, pp.1225-1229, edited by Editorial committee for manual of the Japanese Pharmacopoeia, published by Hirokawa-shoten, Tokyo).

According to Guideline for Hand Hygiene in Health-Care Settings (CDC Guideline: Morbidity and Mortality Weekly Report, 2002; 51 (No.RR-16)) published by Centers for Disease Control and Prevention in the U.S. (CDC), it was reported that chlorhexidine gluconate effectively reduces the number of microorganisms on the skin immediately after rubbed into the skin, and therefore it is a highly active hand antiseptic.

Recently, as the improvement of surgical methods etc. has enabled surgeries for a patient who was conventionally thought to have difficulty having a surgery, more surgeries have required a prolonged surgical time. During such long-time surgeries, there is a possibility that microorganisms flow into surgical sites (patient's body etc.) through pinholes and other damaged parts of rubber gloves worn by surgeons etc. , resulting in microbial infection. Under such circumstances, in the United States, antiseptic-containing handwash products intended for use by healthcare workers are regulated by Division of Over-the-Counter Drug Products in U.S. Food and Drug Administration (FDA), and requirements for in vitro and in vivo testing for handwash products and surgical hand scrubs for healthcare workers are outlined by FDA Tentative Final Monograph for Health-Care Antiseptic Drug Products (Proposed Rules: Federal Register, 1994; 59: 31401-52). In the Monograph, surgical hand scrubs should substantially reduce the number of microorganisms on the intact skin, contain a low-irritant antimicrobial agent, have a broad-spectrum activity, and be fast-acting and persistent. In CDC Guideline, surgical hand scrubs are evaluated for their ability to reduce the number of microorganisms released from hands (1) immediately after hand antisepsis (i.e., immediate activity) (2) after wearing surgical gloves for 6 hours (i.e., persistent activity), and (3) after multiple hand rubs over 5 days (i.e., cumulative activity). Especially, immediate and persistent activities are the most important for evaluating the efficacy of the surgical hand scrubs. Additionally, CDC Guideline (Morbidity and Mortality Weekly Report, 2002; 51 (No.RR-16)) reported that addition of 0.5 or 1.0 mass % chlorhexidine gluconate of the total amount to an alcohol-based preparation resulted in a greater microbicidal effect than alcohol alone.

In Japan, known antiseptics containing chlorhexidine gluconate and an alcohol include a disinfectant composition disclosed by Japanese Patent No. 3515821, containing chlorhexidine, an organic acid and 50% by weight or more ethyl alcohol with the whole system adjusted to a pH of 3 to 4.5, and a viscous quick-drying germicidal disinfectant disclosed by Japanese Patent No. 3592080, comprising a combination of one or more alcohols selected from the group consisting of methyl alcohol, ethyl alcohol and isopropyl alcohol; a germicidal disinfectant such as chlorhexidine gluconate; and a hydrophobic polymer such as hydrophobized hydroxypropyl methyl cellulose, in which a stearyloxy hydroxypropoxyl group is introduced, the hydrophobic polymer being present in an amount from 0.1 to 20% by weight of the total weight. Either of them is, however, an alcohol-based antiseptic, and unfortunately it causes skin irritation induced by alcohol and skin damage on hands or other parts to which the antiseptic is applied. Further, it only provides an inadequate effect in the above-described persistent activity in reality.

DISCLOSURE OF THE INVENTION

PROBLEMS TO BE SOLVED BY THE INVENTION

The object of the present invention is to provide a microbicidal and antiseptic ointment having a fast-acting and prolonged persistent antiseptic activity, for use on human hands or skin without alcohol-induced skin irritation and damage.

MEANS FOR SOLVING THE PROBLEMS

The inventors have intensively carried out investigations to achieve the above-mentioned object and finally found that an ointment, in which at least one kind selected from the group consisting of chlorhexidine and/or a salt thereof is present in an amount from about 0.1 to 4 mass %, and a hydrocarbon alcohol having 2 to 3 carbon atoms is present in an amount from about 8 to 25 mass %, demonstrates a fast-acting and persistent antiseptic effect and prevents alcohol-induced skin irritation and skin damage on hands or the like. The inventors have studied further and accomplished the present invention.

The present invention relates to:

(1) a microbicidal and antiseptic ointment, comprising at least one kind selected from the group consisting of chlorhexidine and a salt thereof in an amount from 0.1 to 4 mass % and a hydrocarbon alcohol having 2 to 3 carbon atoms in an amount from 8 to 25 mass %, wherein the ointment sustains a microbicidal effect for at least 6 hours, the microbicidal effect being approved by a test method for evaluation of hand antisepsis;

(2) the microbicidal and antiseptic ointment of the above-mentioned (1), wherein the test method for evaluation of hand antisepsis is a glove juice method, a palm stamp method or a finger streak plate method;

(3) the microbicidal and antiseptic ointment of the above-mentioned (1), wherein the ointment is an emulsion ointment;

(4) the microbicidal and antiseptic ointment of the above-mentioned (3), wherein the emulsion ointment comprises (a) at least one oily base selected from the group consisting of a lipid, higher alcohol and higher fatty acid compound; (b) a nonionic surfactant; and (c) at least one aqueous base selected from the group consisting of a water-soluble polyhydric alcohol and water;

(5) the microbicidal and antiseptic ointment of the above-mentioned (4), wherein the higher fatty acid compound is a higher fatty acid, higher fatty acid salt, higher fatty acid ester or higher fatty acid amide;

(6) a method for disinfecting a human hand or skin, comprising applying to the human hand or skin an ointment which comprises at least one kind selected from the group consisting of chlorhexidine and a salt thereof in an amount from 0.1 to 4 mass % and a hydrocarbon alcohol having 2 to 3 carbon atoms in an amount from 8 to 25 mass %, and sustains a microbicidal effect for at least 6 hours, the microbicidal effect being approved by a test method for evaluation of hand antisepsis;

(7) a use of an ointment as a microbicidal and antiseptic ointment, the ointment comprising at least one kind selected from the group consisting of chlorhexidine and a salt thereof in an amount from 0.1 to 4 mass % and a hydrocarbon alcohol having 2 to 3 carbon atoms in an amount from 8 to 25 mass %, wherein the ointment sustains a microbicidal effect for at least 6 hours, the microbicidal effect being approved by a test method for evaluation of hand antisepsis;

(8) a microbicidal and antiseptic ointment, comprising at least one kind selected from the group consisting of chlorhexidine and a salt thereof in an amount from 0.1 to 4 mass % and a hydrocarbon alcohol having 2 to 3 carbon atoms in an amount from 8 to 25 mass %;

(9) a method for disinfecting a human hand or skin, comprising applying to the human hand or skin an ointment which comprises at least one kind selected from the group consisting of chlorhexidine and a salt thereof in an amount from 0.1 to 4 mass % and a hydrocarbon alcohol having 2 to 3 carbon atoms in an amount from 8 to 25 mass %;

(10) a use of an ointment as a microbicidal and antiseptic ointment, the ointment comprising at least one kind selected from the group consisting of chlorhexidine and a salt thereof in an amount from 0.1 to 4 mass % and a hydrocarbon alcohol having 2 to 3 carbon atoms in an amount from 8 to 25 mass %;

(11) a use of an ointment in the manufacture of a microbicidal and antiseptic ointment, the ointment comprising at least one kind selected from the group consisting of chlorhexidine and a salt thereof in an amount from 0.1 to 4 mass % and a hydrocarbon alcohol having 2 to 3 carbon atoms in an amount from 8 to 25 mass %, wherein the ointment sustains a microbicidal effect for at least 6 hours, the microbicidal effect being approved by a test method for evaluation of hand antisepsis; and

(12) a use of an ointment in the manufacture of a microbicidal and antiseptic ointment, the ointment comprising at least one kind selected from the group consisting of chlorhexidine and a salt thereof in an amount from 0.1 to 4 mass % and a hydrocarbon alcohol having 2 to 3 carbon atoms in an amount from 8 to 25 mass %.

EFFECT OF THE INVENTION

According to the present invention, the microbicidal and antiseptic ointment provides a significant fast-acting microbicidal effect and further sustains the effect for a prolonged period of time, even at least 6 hours after disinfection. Due to the persistent microbicidal effect over at least 6 hours, the microbicidal and antiseptic ointment can inhibit the flow of microorganisms into the surgical sites through, if any, pinholes and other damage parts of rubber gloves used by surgeons etc. during a long-time surgery.

The microbicidal and antiseptic ointment of the present invention, which contains an alcohol in an extremely lower than customary concentration for the purpose of microbicidal disinfection (usually about 85 mass %), can inhibit alcohol-induced irritation and prevent skin damage caused by frequent use of microbicidal agents from health-care workers. Furthermore, since the microbicidal and antiseptic ointment of the present invention can be used for microbicidal or antiseptic treatment of affected areas such as injuries and burns, and even sustain the prolonged microbicidal and antiseptic effect, the ointment can prevent the onset of infectious diseases and the like in the affected areas.

BRIEF DESCRIPTION OF DRAWINGS

Fig.1 shows hand-shaped media incubated at 35°C for 18 hours, after palms were pushed against the media at 3- and 6-hour post-disinfection with preparation 1. Each value in parenthesis in the figure represents the number of colonies.

Fig.2 shows hand-shaped media incubated at 35°C for 18 hours, after palms were pushed against the media at 3- and 6-hour post-disinfection with preparation 2. Each value in parenthesis in the figure represents the number of colonies.

Fig.3 shows hand-shaped media incubated at 35°C for 18 hours, after palms were pushed against the media at 3- and 6-hour post-disinfection with reference preparation 1. Each value in parenthesis in the figure represents the number of colonies.

Fig.4 shows hand-shaped media incubated at 35°C for 18 hours, after palms were pushed against the media at 3- and 6-hour post-disinfection with reference preparation 3. Each value in parenthesis in the figure represents the number of colonies.

Fig.5 shows a time-course graph of the residual ratio of microbial count in the palm after disinfection.

BEST MODE FOR CARRYING OUT THE INVENTION

(I) First embodiment of the microbicidal and antiseptic ointment according to the present invention

In a first embodiment, the present invention provides a microbicidal and antiseptic ointment containing at least one kind selected from the group consisting of chlorhexidine and a salt thereof in an amount from about 0.1 to 4 mass %, and a hydrocarbon alcohol having 2 to 3 carbon atoms in an amount from about 8 to 25 mass %. The microbicidal and antiseptic ointment also sustains the microbicidal effect for at least 6 hours, which is approved by test methods for evaluation of hand antisepsis.

Chlorhexidine and a salt thereof

Chlorhexidine used for the present invention is a known chemical compound, which was first developed in U.K. in 1954 and originally named as 1,1-hexamethylene-bis[5-(4-chlorophenyl)biguanide]. The compound has a wide-spectrum antibacterial activity, namely it exhibits a microbicidal and antiseptic effect against a wide range of bacteria. Examples of the chlorhexidine salt include a mineral or organic salt. To be more specific, hydrochloride, gluconate, acetate or the like is preferred. Most preferred salt is gluconate (chlorhexidine gluconate). Chlorhexidine and a salt thereof can be used alone or in combination of two or more.

In the microbicidal and antiseptic ointment of the present invention, chlorhexidine and/or a salt thereof is present in amount of about 0.1 to 4 mass %, preferably about 0.2 to 4 mass %, more preferably about 0.2 to 3 mass %, even more preferably about 0.2 to 2 mass %, and most preferably about 0.2 to 1 mass % of the total amount of the ointment. When the amount of chlorhexidine and/or a salt thereof present in the ointment is within the above range, the ointment can not only exhibit a fast-acting microbicidal effect, but also sustain the microbicidal effect approved by test methods for evaluation of hand antisepsis, for a prolonged period of time, specifically, at least 6 hours. Additionally, the ointment can inhibit irritation to skin or the like.

Lower hydrocarbon alcohol

Examples of the hydrocarbon alcohol having 2 to 3 carbon atoms (sometimes hereinafter referred to just as alcohol) include ethanol, propanol, isopropanol, or the like. Ethanol or isopropanol is preferred. The hydrocarbon alcohol having 2 to 3 carbon atoms can be used alone or as a mixture of two or more of them. Methylated alcohol or the like can be also mixed into the aforementioned alcohol. Addition of the alcohol to an ointment containing chlorhexidine and/or a salt thereof can further expand the antibacterial spectrum shown by the microbicidal and antiseptic ointment of the present invention. In the microbicidal and antiseptic ointment of the present invention, the amount of the alcohol preferably ranges as much as it enables the ointment to maintain an ointment form and the antibacterial (microbicidal) effect. The amount of the alcohol usually ranges about 8 to 25 mass %, preferably about 10 to 20 mass %. When the amount of the alcohol is within the above range, the microbicidal and antiseptic ointment of the present invention can exhibit a significant microbicidal and antiseptic effect and sustain the effect for a prolonged period of time.

Ointment base

According to the present invention, the microbicidal and antiseptic ointment can be provided in the form of a gel or emulsion ointment, or the like. A preferred form is an emulsion ointment because of its low-irritancy and highly preventive effect on skin damage of hands. The emulsion ointment includes water-in-oil (W/O), oil-in-water (O/W), W/O/W, O/W/O, liquid crystalline types, or the like. Specifically, in one example of the W/O emulsion ointment, fine particles of an aqueous phase are uniformly dispersed in an oily-based phase. In one example of the O/W emulsion ointment, fine particles of oily phase are uniformly dispersed in an aqueous phase. In one example of the W/O/W emulsion ointment, a type of multiple emulsions, oil-droplets in which extremely fine particles of an aqueous phase are enclosed by an oily phase are dispersed in an aqueous phase. In one example of the O/W/O emulsion ointment, a type of multiple emulsions, water-droplets in which extremely fine particles of an oily phase are enclosed by an aqueous phase are dispersed in an oily phase. In one example of the liquid crystalline emulsion ointment, lipids therein can form a lipid bilayer on the skin, which captures moisture between the layers and thereby forms a lamellar structure (membrane with layered structure). The lamellar structure includes multilamellar (lipid-dispersed type) and aqua-lamellar (moisture-holding structure) structures. It is preferred that the microbicidal and antiseptic ointment has a lamellar structure, but the present invention is not limited thereto. Additionally, the emulsion ointment includes a cream.

In the microbicidal and antiseptic ointment of the present invention, an eligible base for use in the emulsion ointment may be a base containing an oily base, aqueous base and nonionic surfactant. Examples of the oily base include, but are not limited to, a lipid, a higher alcohol, a higher fatty acid compound, or the like. The oily base such as a lipid, higher alcohol or higher fatty acid compound can be used alone or in combination of two or more. An example of the combination includes a lipid and a higher alcohol, a lipid and a higher fatty acid compound, a higher alcohol and a higher fatty acid compound, or a lipid, higher alcohol and higher fatty acid compound. The combination of a lipid, higher alcohol and higher fatty acid compound is preferred.

Examples of the lipid used in the oily base include, but are not limited to, a hydrocarbon such as liquid paraffin (mineral oil), heavy liquid isoparaffin, light liquid isoparaffin, $\alpha$-olefin oligomer, polyisobuten, hydrogenated polyisobuten, polybuten, squalane, olive-derived squalane, squalene, petrolatum, solid paraffin, etc.; a wax such as candelilla wax, carnauba wax, rice wax, Japan wax, beeswax, montan wax, ozokerite, ceresine wax, paraffin wax, microcrystalline wax, petrolatum, Fischer-Tropsch wax, polyethylene wax, ethylene-propylene copolymer, etc.; a vegetable oil or fat such as coconut oil, palm oil, palm kernel oil, hydrogenated palm oil, safflower oil, olive oil, castor oil, avocado oil, sesame oil, tea oil, evening primrose oil, wheat germ oil, macadamia nut oil, hazelnut oil, kukui nut oil, rosehip oil, meadowfoam oil, persic oil, tea tree oil, menthe oil, corn oil, rape seed oil, sunflower oil, wheat germ oil, flaxseed oil, cotton seed oil, soybean oil, peanut oil, rice bran oil, cocoa butter, shea butter, hydrogenated coconut oil, hydrogenated castor oil, jojoba oil, hydrogenated jojoba oil, etc.; a derivative of a vegetable oil or fat such as coconut oil triglyceride, palm oil triglyceride, etc.; an animal oil or fat such as beef tallow, milk fat, horse fat, egg yolk oil, mink oil, turtle oil, etc.; an animal wax such as spermaceti, lanolin, orange roughy oil, etc.; a lanolin such as liquid lanolin, hydrogenated lanolin, purified lanolin, acetylated lanolin, liquid acetylated lanolin, hydroxylated lanolin, polyoxyethylene (hereinafter referred to as POE) lanolin, lanolin fatty acid, hard lanolin fatty acid, etc.; a phospholipid such as lecithin, phosphatidyl choline, phosphatidylethanolamine, phosphatidylserine, phosphatidylglycerol, phosphatidylinositol, a sphingolipid such as sphingomyelin etc., phosphatidic acid, lysolecithin, etc.; a phospholipid derivative such as hydrogenated soybean phospholipid, partially hydrogenated soybean phospholipid, hydrogenated egg yolk phospholipid, partially hydrogenated egg yolk phospholipid, etc.; a lipid complex such as phospholipid-cholesterol complex, phospholipid-phytosterol complex, etc.; a silicone such as dimethicone (dimethylpolysiloxane), dimethicone with a high degree of polymerization (dimethylpolysiloxane with a high degree of polymerization), cyclomethicone (cyclodimethylsiloxane, decamethyl cyclopentasiloxane, octamethyl cyclotetrasiloxane), phenyltrimethicone, diphenyldimethicone, phenyldimethicone, stearoxypropyldimethylamine, (aminoethylaminopropyl methicone/dimethicone) copolymer, dimethiconol, dimethiconol crosspolymer, silicone resin, silicone gum, amino-modified silicone such as aminopropyl dimethicone, amodimethicone, etc., cation-modified silicone, polyether-modified silicone such as dimethicone copolyol etc. , polyglycerin-modified silicone, sugar-modified silicone, carboxylated silicone, phosphated silicone, sulfated silicone, alkyl-modified silicone, fatty acid-modified silicone, alkylether-modified silicone, amino acid-modified silicone, peptide-modified silicone, fluorine-modified silicone, cation- and polyether-modified silicone, amino- and polyether-modified silicone, alkyl- and polyether-modified silicone, polysiloxane-oxyalkylene copolymer, etc.; a fluorine oil such as perfluorodecane, perfluorooctane, perfluoro polyether, etc.; or the like.

Examples of the higher alcohol used in the oily base include, but are not limited to, cetyl alcohol, myristyl alcohol, oleyl alcohol, lauryl alcohol, cetostearyl alcohol, stearyl alcohol, arachyl alcohol, behenyl alcohol, jojoba alcohol, chimyl alcohol, selachyl alcohol, batyl alcohol, hexyldecanol, isostearyl alcohol, 2-octyldodecanol, dimerdiol, cholesterol, dihydrocholesterol, lanosterol, dihydrolanosterol, sitosterol, decyltetradecanol, phytosterol, lanolin alcohol, hydrogenated lanolin alcohol, etc. Inter alia, preferred is a higher alcohol having about 7 to 25 carbon atoms, more preferred is a higher alcohol having about 12 to 22 carbon atoms, because of easier emulsifying and stabilizing the ointment. Such a higher alcohol can be used alone or in combination two or more.

Examples of the higher fatty acid compound used in the oily base include a higher fatty acid, higher fatty acid salt, higher fatty acid ester or higher fatty acid amide. An example of the higher fatty acid is a saturated or unsaturated and straight-or branched-chain higher fatty acid, specifically, including caprylic acid, pelargonic acid, capric acid, undecyl acid, lauric acid, tridecyl acid, myristic acid, pentadecyl acid, palmitic acid, heptadecyl acid, stearic acid, isostearic acid, nonadecanoic acid, arachic acid, behenic acid, undecylenic acid, oleic acid, elaidic acid, cetoleic acid, erucic acid, linoleic acid, linolenic acid, stearolic acid, sebacic acid, 12-hydroxystearic acid, palmitoleic acid, docosahexaenoic acid, eicosapentaenoic acid, isohexadecanoic acid, anteiso-henicosanoic acid, branched long-chain fatty acid, dimeric acid, hydrogenated dimeric acid or a salt thereof, but they are not limited thereto. Inter alia, preferred is a higher fatty acid having 10 to 25 carbon atoms, and more preferred is a saturated higher fatty acid having 14 to 22 carbon atoms. An example of the higher fatty acid salt is an aluminum, calcium, magnesium, zinc or potassium salt, etc.

Examples of the higher fatty acid ester include, but are not limited to, octyldodecyl myristate, hexyldecyl myristate, octyldodecyl isostearate, cetyl palmitate, octyldodecyl palmitate, cetyl octanoate, hexyldecyl octanoate, octyldodecyl neodecanoate, oleyl oleate, octyldodecyl oleate, octyldodecyl ricinoleate, octyldodecyl lanolate, hexyldecyl dimethyloctanoate, octyldodecyl erucate, hydrogenated castor oil isostearate, ethyl oleate, ethyl avocadate, isopropyl myristate, isopropyl palmitate, octyl palmitate, isopropyl isostearate, isopropyl lanolate, diethyl sebacate, diisopropyl sebacate, dioctyl sebacate, diisopropyl adipate, dibutyloctyl sebacate, cholesteryl stearate, cholesteryl isostearate, cholesteryl oleate, di(cholesteryl/behenyl/ octyldodecyl) N-lauroyl-L-glutamate, di(cholesteryl/octyldodecyl) N-lauroyl-L-glutamate, di(phytosteryl/behenyl/octyldodecyl) N-lauroyl-L-glutamate, di(phytosteryl/octyldodecyl) N-lauroyl-L-glutamate, acyl sarcosine alkyl esters such as isopropyl N-lauroyl sarcosinate, cholesteryl 12-hydroxystearate, cholesteryl macadamiate, phytosteryl macadamiate, phytosteryl isostearate, soft lanolin fatty acid cholesterol ester, hard lanolin fatty acid cholesterol ester, branched long-chain fatty acid cholesterol ester, long-chain $\alpha$-hydroxyalkyl acid cholesterol ester, diisopropyl dimer dilinoleate, diisostearyl dimer dilinoleate, di(isostearyl/phytosteryl) dimer dilinoleate, (phytosteryl/behenyl) dimer dilinoleate, (phytosteryl/isostearyl/cetyl/stearyl/behenyl) dimer dilinoleate, dimer dilinoleyl dimer dilinoleate, dimer dilinoleyl diisostearate, dimer dilinoleyl hydrogenated rosinate, hydrogenated castor oil dimer dilinoleate, hydroxyalkyl dimer dilinoleyl ether, etc.

Examples of the higher fatty acid amide include, but are not limited to, oleamide, stearamide, erucamide, behenamide, N-oleylpalmitamide, N-stearylerucamide, etc. In addition, amides include an alkanolamide.

Examples of the higher fatty acid alkanolamide include, but are not limited to, coconut fatty acid monoethanolamide (cocamide MEA), coconut fatty acid diethanolamide (cocamide DEA), lauric monoethanolamide (lauramide MEA), lauric diethanolamide (lauramide DEA), lauric monoisopropanolamide (lauramide MIPA), palmitic monoethanolamide (palmitamide MEA), palmitic diethanolamide (palmitamide DEA), coconut fatty acid methyl ethanolamide (cocamide methyl MEA), etc.

Such a higher fatty acid compound can be used alone or in combination of two or more.

The proportion of the oily base is preferably about 0.5 to 50 mass % of the total amount of the final preparation in an emulsion ointment form of the microbicidal and antiseptic ointment according to the present invention. When a lipid, a higher alcohol and a higher fatty acid compound are used in combination as the oily base, the proportions are, but are not limited to, preferably about 3 to 97 mass % lipid, about 2 to 33 mass % higher alcohol and about 1 to 13 mass % higher fatty acid compound of the total amount of the oily base.

Examples of the aqueous base include, but are not limited to, a water-soluble polyhydric alcohol, water, or the like.

Examples of the water-soluble polyhydric alcohol include, but are not limited to, an alcohol having two or more hydroxy groups within a molecule, specifically, including glycerol, xylitol, d-sorbitol, ethylene glycol, diethylene glycol, triethylene glycol, dipropylene glycol, butylene glycol, 1,3-butylene glycol, propylene glycol, hexylene glycol, polyethylene glycol 200 to 600, polyethylene glycol 1000 to 4000, methyl gluceth, maltitol, mannitol, glucose, sorbitol, 3-methyl-1,5-pentanediol, 2,4-diethyl-1,5-pentanediol, 2-butyl-2-ethyl-1,3-propanediol, 2,2-diethyl-1,3-propanediol, 2-methyl-2-propyl-1,3-propanediol, 2-ethyl-2-sec-butyl-1,3-propanediol, 2,2,4-trimethyl-1,3-pentanediol, 2,4-dimethyl-1,5-pentanediol, 3,3-dimethyl-1,5-pentanediol, etc. Such a polyhydric alcohol can be used alone or in combination of two or more.

The proportion of the aqueous base is usually about 1 to 30 mass %, preferably about 5 to 20 mass % of the total amount of the ointment of the present invention. The proportion of moisture, except for an oily ingredient, chlorhexidine gluconate, ethanol and a surfactant, etc., is preferably about 10 to 90 mass % of the total amount of the ointment.

Examples of the water include, but are not limited to, ordinary water, purified water, hard water, soft water, natural water, deep sea water, alkaline ionized water produced by electrolysis, acidic ionized water produced by electrolysis, ionized water, clustered water, etc. Such water can be used alone or in combination of two or more.

Examples of the nonionic surfactant used in the present invention include, but are not limited to, a propylene glycol fatty acid ester such as propylene glycol monostearate etc.; a glycerol fatty acid ester such as glyceryl monostearate, glyceryl distearate, glyceryl monooleate, glyceryl dioleate, glyceryl tri-2-ethylhexanoate, etc.; a polyglycerol fatty acid ester such as diglyceryl monostearate, diglyceryl monooleate, diglyceryl monoisostearate, diglyceryl dioleate, diglyceryl diisostearate, hexaglyceryl pentastearate, hexaglyceryl tristearate, etc.; a POE glycerol fatty acid ester such as POE glyceryl monostearate etc.; a sorbitan fatty acid ester such as sorbitan monopalmitate, sorbitan monostearate, sorbitan monosesquistearate, etc.; a POE sorbitan fatty acid ester such as POE 20 mol sorbitan monolaurate etc.; a POE sorbitol fatty acid ester such as POE 6 mol sorbitol hexastearate etc.; a POE hydrogenated castor oil such as POE 20 to 60 mol hydrogenated castor oil etc.; a POE sterol/hydrogenated sterol; a POE glycol fatty acid ester; a POE alkyl ether; a POE polyoxypropylene alkyl ether; a POE alkyl phenyl ether; a sucrose fatty acid ester such as sucrose stearate etc.; a propylene glycol fatty acid ester such as propylene glycol monostearate etc.; a pentaerythritol fatty acid ester such as pentaerythritol monostearate etc.; a decaglycerol fatty acid ester such as decaglyceryl monostearate, decaglyceryl monooleate, decaglyceryl monoisostearate, etc.; a POE polyethylene glycol fatty acid ester such as POE 10 to 55 mol polyethylene glycol monostearate, POE 10 mol polyethylene glycol monooleate, etc.; a POE glycerol fatty acid ester such as POE 15 mol glyceryl monostearate, POE 15 mol glyceryl monooleate, etc.; a POE alkyl ether such as POE 2 to 10 mol oleyl ether etc. ; POE 5 to 20 mol cholesteryl ether; POE 5 to 30 mol phytosterol; or the like.

Such a nonionic surfactant can be used alone or in combination of two or more.

A lipophilic nonionic surfactant used as the nonionic surfactant in the ointment preferably has an HLB of 7 or less. In addition, the lipophilic nonionic surfactant is preferably in a semi-solid or solid form at room temperature. As the lipophilic nonionic surfactant, a glycerol fatty acid ester is particularly preferred.

The proportion of the lipophilic nonionic surfactant used as the nonionic surfactant is usually about 0.1 to 10 mass %, preferably about 0.5 to 5 mass % of the total amount of the ointment.

A hydrophilic nonionic surfactant used as the nonionic surfactant in the ointment preferably has an HLB of 10 or more. The hydrophilic nonionic surfactant is preferably a POE polyethylene glycol fatty acid ester, POE cholesteryl ether or POE hydrogenated castor oil. Particularly preferred is a POE cholesteryl ether or a POE hydrogenated castor oil.

The proportion of the hydrophilic nonionic surfactant used as the nonionic surfactant in the ointment is usually about 1 to 15 mass %, preferably about 2 to 10 mass % of the total amount of the ointment.

The nonionic surfactant used in the present invention can be either lipophilic or hydrophilic, or a combination of lipophilic and hydrophilic one. The combination thereof is preferred, and inter alia, the combination of a glycerol fatty acid ester and a POE hydrogenated castor oil or a POE cholesteryl ether is particularly preferred.

In present invention, any other surfactant such as an anionic, amphoteric or cationic surfactant can be used in combination with the nonionic surfactant.

In an emulsion form of the ointment according to the present invention, the proportion of the oily base to the nonionic surfactant (mass ratio, oily base: nonionic surfactant) is preferably about 1:1 to 3:1.

In the microbicidal and antiseptic ointment of the present invention, an eligible base for use in a gel ointment includes polyacrylamide, polyvinyl alcohol, polyethylene oxide, polypropylene oxide, polyester, polyacrylate, gelatin, collagen, polylactic acid, polyglycolic acid, a synthetic polymer such as polylactic acid-polyglycolic acid copolymer, alginic acid, an alginate salt, a polysaccharide such as agarose, chitin, chitosan, hyaluronan, starch or pectin, a polypeptide such as poly-γ-glutamic acid, poly-L-lysine or polyarginine, a polyanhydride, a copolymer or derivative thereof, or for example, a silicone gel composition described in JP-A No. 2002-179919.

In addition to the above-mentioned ointment base, a customary excipient to be incorporated into an ointment may be optionally incorporated into the microbicidal and antiseptic ointment of the present invention, as long as the object of the present invention can be achieved. Examples of the excipient include, but are not limited to, a moisturizer, thickener, antioxidant, antiseptic, chelating agent, pH adjuster, fragrance, ultraviolet absorber or refrigerant, etc.

Examples of the moisturizer include, but are not limited to, natural moisturizing factor (NMF); an amino acid, a carboxylic acid or an ester thereof, or lactic acid, all of which are NMF components; pyrrolidone carboxylic acid; a glycosaminoglycan such as hyaluronan, chondroitin sulfate, etc.; a polyhydric alcohol such as glycerin, propylene glycol, butylene glycol, sorbit, or the like; a hydrolysate of a protein such as soluble collagen, elastin, keratin, etc.; placenta extract; mucin; betaine; chitin or chitosan; bifidobacterium metabolite or yeast metabolite through fermentation; yeast extract; or the like.

Examples of the thickener include, but are not limited to, carboxyvinyl polymer, carboxymethyl cellulose, hydroxyethyl cellulose, hydroxypropyl cellulose, polyvinyl alcohol, polyvinyl pyrrolidone, guar gum, xanthan gum, gelatin, or the like.

Examples of the antioxidant include, but are not limited to, tocopherol (Vitamin E), a tocopherol derivative such as tocopherol acetate etc.; BHT or BHA; a gallic acid derivative such as propyl gallate etc.; Vitamin C (ascorbic acid) and/or a derivative thereof; erythorbic acid and a derivative thereof; a sulfite salt such as sodium sulfite etc.; a bisulfite salt such as sodium bisulfite etc.; or the like.

Examples of the antiseptic include, but are not limited to, a paraben (methylparaben, ethylparaben, propylparaben, butylparaben, etc.), salicylic acid, sodium benzoate, phenoxyethanol, sorbic acid, benzalkonium chloride, benzethonium chloride, hinokitiol, or the like.

Examples of the chelating agent include, but are not limited to, an edetate (ethylenediaminetetraacetate) such as EDTA, EDTA2Na, EDTA3Na, EDTA4Na, etc.; a hydroxyethyl ethylenediamine triacetate such as HEDTA3Na etc.; a pentetate (diethylenetriamine pentaacetate); phytic acid; a phosphonic acid such as etidronic acid etc. and a salt thereof such as a sodium salt; sodium oxalate; or the like.

Examples of the pH adjuster include, but are not limited to, citric acid, sodium citrate, lactic acid, sodium lactate, glycolic acid, succinic acid, acetic acid, sodium acetate, malic acid, tartaric acid, fumaric acid, phosphoric acid, hydrochloric acid, sulfuric acid, monoethanolamine, diethanolamine, triethanolamine, isopropanolamine, triisopropanolamine, 2-amino-2-methyl-1,3-propanediol, 2-amino-2-hydroxymethyl-1,3-propanediol, arginine, sodium hydroxide, potassium hydroxide, ammonia solution, guanidine carbonate, ammonium carbonate, or the like.

Examples of the fragrance include, but are not limited to, various kinds of an essential oil such as camphor, carrot seed oil, clove oil, methyl cinnamate, geraniol, L-menthol, L-menthone, eucalyptus oil, lime oil, lavender oil, D-limonene, linalool, liral, lilial, lemon oil, rose oil, rosemary oil, or the like.

Examples of the ultraviolet absorber include, but are not limited to, 2-ethylhexyl(octyl) paramethoxy cinnamate, 2-ethylhexyl(octyl) paradimethyl aminobenzoate, oxybenzone (benzophenone-3), 2-ethylhexyl(octyl) salicylate, 4-tert-butyl-4-methoxy-benzoylmethane, or the like.

Examples of the refrigerant include, but are not limited to, 1-menthol, camphor, or the like.

Additionally, any other medicative ingredient may be incorporated into the microbicidal and antiseptic ointment of the present invention, as long as the microbicidal and antiseptic effect of chlorhexidine gluconate is not interfered. Examples of the other medicative ingredient include, but are not limited to, any other microbicidal drug such as acrinol, alkylpolyaminoethyl glycine, isopropylmethyl phenol, cetylpyridinium chloride, decalinium chloride, berberine chloride, benzalkonium chloride, cetrimide, resorcin, etc.; an anti-inflammatory drug such as hydrocortisone acetate, hydrocortisone, prednisolone acetate, prednisolone, etc.; a topical anesthetic such as dibucaine hydrochloride, procaine hydrochloride, lidocaine hydrochloride, methylephedrine hydrochloride, etc; an antihistamine drug such as diphenhydramine hydrochloride, chlorpheniramine maleate, etc; or the like. Furthermore, one or more known drugs such as a vasoconstrictor, protectant, anti-irritant or analgesic, etc. may be optionally incorporated into the microbicidal and antiseptic ointment of the present invention.

Persistency of microbicidal effect

According to the first embodiment of the present invention, the microbicidal and antiseptic ointment sustains the microbicidal effect for at least 6 hours, which is approved by test methods for evaluation of hand antisepsis. The test methods for evaluation of hand antisepsis include a glove juice test, palm stamp method and finger streak plate method. An ointment showing the microbicidal effect approved by any of the glove juice test, palm stamp method or finger streak plate method, is included in ointments with microbicidal activity. An ointment sustaining the microbicidal effect approved by any of the above-mentioned methods for at least 6 hours is defined as the microbicidal and antiseptic ointment according to the present invention. Most preferred is an ointment sustaining the microbicidal effect for at least 6 hours, which is approved by the palm stamp method.

Glove juice test

A glove juice test is recommended by FDA as an effective test method for evaluation of handwash products and surgical hand scrubs for healthcare workers. The glove juice test is described in Geka Shinryo, Apr: 513-518 (1982) and FDA proposed rules on Over-the-Counter (OTC) topical antimicrobial drug products in Federal Register, 39, (179): 33137 (1974), Federal Register, 43, (4): 1242 (1978), and Federal Register, 59, (116): 31444 (1994). The test substantially consists of the measurement of the baseline value and the main test (a method for evaluating the efficacy of hand antisepsis).

The baseline is measured as the following procedures. After subject selection, each selected subject washes his/her hands with soap, put a sterile glove on his/her hand, pour into the glove 50 ml of a sampling solution (prepared by dissolving 0.4 g of potassium hydrogen phosphate, 10.1 g of disodium hydrogen phosphate and 1 g of TritonX-100 in 1.0 L of distilled water and adjusting a pH to 7.8) and massage the glove-clad hand for one minute, and then take a certain volume (1 ml) of the sampling solution out of the glove. Next, a diluted sampling solution is incubated and thereafter colonies are counted and microbial count in 50 ml of the sampling solution is calculated. With regard to dilution, the collected sampling solution is poured into a sterile test tube containing an equal volume of a neutralizer (for example, prepared by dissolving 10 g of Tween80 and 3 g of lecithin in 100 ml of distilled water). After vigorously mixing, the mixture (hereinafter referred to as a concentrate solution) is diluted, for example 10, 100 and 1000 times with a diluent so that colonies can be counted. A diluent that can be used may be prepared by dissolving 26.22 g of potassium hydrogen phosphate and 7.78 g of sodium carbonate in 1000 ml of distilled water. With regard to incubation, for example, a concentrate or diluted solution is spread on an agar medium (for example, TSA (Trypticase Soy Agar) plate etc.) with a Conradi stick or the like and the plate is incubated at 30°C for 24 hours. After incubation, colonies are counted and microbial count in 50 ml of the sampling solution is calculated. The measurement is, for example, performed twice and an average value of the counts is calculated. The average value is referred to as a baseline value for each subject (microbial count before disinfection).

The main test is a method to evaluate the microbicidal effect immediately or an appropriate time after disinfection. To be more specific, after surgical handwash with a gel composition of the present invention, sterile gloves are put on hands. Immediately following this, 50 ml of the same sampling solution as used for the measurement of a baseline value is poured into one of the gloves. Like the measurement of a baseline value, the glove-clad hand is massaged for one minute and then a certain volume (1 ml) of the sampling solution is taken out of the glove (hereinafter this step is referred to as sampling). Like the measurement of a baseline value, the resulting sampling solution is diluted, incubated, thereafter colonies are counted and microbial count in 50 ml of the sampling solution is calculated. Thus, the total microbial count in one hand can be determined. Sampling from the other hand is performed in the same manner as described above, not immediately, but 6 hours after the gloves are put on the hand. The resulting sampling solution is diluted, the diluted solution is incubated, and thereafter, total microbial count in the other hand is determined.

The antiseptic efficacy (evaluation of antiseptic effect) is determined by calculating a reduction ratio from the microbial counts before and after disinfection and then comparing microbicidal effects. Specifically, the comparison is usually performed using a log reduction factor of the microbial count (RF value). RF value is calculated by the following formula (1).

```
Formula (1)

RF value = log (microbial count before disinfection)-log

(microbial count after disinfection)

= log (microbial count before disinfection/ microbial count

after disinfection) (1)
```

When RF value is 1.2 or more, the microbicidal effect is approved by the present invention. Preferred RF value is 1.5 or more. The main test, in which sampling is performed from one hand at a time after both hands are disinfected, enables the microbicidal effects to be evaluated at different times after disinfection.

Palm stamp method

In a palm stamp method, instead of the sampling step in the above-described glove juice method, a whole palm is pushed against a hand-shaped medium (for example, SCD medium; SCDLP medium for use with palms; Palm Stamp Check (manufactured by Nikken bio medical laboratory); Hand Petan Check (trademark registered in Japan by Eiken Kizai Co., LTD.), etc.), immediately or an appropriate time after both hands are disinfected with the microbicidal and antiseptic ointment. Thus, microbial sampling can be performed. The hand-shaped medium is incubated at about 35°C for about 18 hours, and then microbial count is measured for evaluation of the antiseptic effect. The microbial count in a palm before disinfection is measured in the same manner as described above and is referred to as a baseline value (microbial count before disinfection).

The evaluation of the antiseptic effect is performed using a reduction ratio of the microbial count after disinfection relative to the corresponding count before disinfection. When the reduction ratio obtained by the following formula (2), is 80% or more, the microbicidal effect is approved.

Formula (2)

Reduction ratio = (microbial count before disinfection - microbial count after disinfection)/ (microbial count before disinfection) x 100 (2)

The palm stamp test, in which microbial sampling is performed by pushing one hand against a hand-shaped medium at a time after both hands are disinfected, enables the microbicidal effects to be evaluated at different times after disinfection. When the reduction ratio 6 hours after disinfection is 80% or more in the palm stamp method, the 6-hour prolonged microbicidal effect of the present invention is approved.

Finger streak plate method

In a finger streak plate method, instead of the sampling step described in the above-mentioned glove juice method, microbial sampling is performed as the following procedures. Immediately or an appropriate time after both hands are disinfected with the microbicidal and antiseptic ointment, a subject places his/her three fingers, neither first nor fifth fingers, on an agar medium for use in the measurement of viable bacteria (for example, SCDLP medium etc.), moves the fingers toward his/her while scraping the surface of the medium, and marks with three parallel streaks. Thus, microbial sampling can be performed. The medium streaked by the fingers is incubated at about 35 °C for about 18 hours, and then microbial count is measured for evaluation of the antiseptic effect. The microbial count before disinfection on three fingers, neither first nor fifth fingers, is also measured in the same manner as described above, and is referred to as a baseline value (microbial count before disinfection).

The evaluation of the antiseptic effect is performed using a reduction ratio of the microbial count after disinfection relative to the corresponding count before disinfection. When the reduction ratio obtained by the previous formula (2), is 80% or more, the microbicidal effect is approved.

The finger streak plate method test, in which microbial sampling is performed from one hand at a time by streaking an agar medium for use in the measurement of viable bacteria after both hands are disinfected, enables the microbicidal effects to be evaluated at different times after disinfection. When the reduction ratio 6 hours after disinfection is 80% or more in the finger streak plate method, the 6-hour prolonged microbicidal effect of the present invention is approved.

(II) Second embodiment of the microbicidal and antiseptic ointment according to the present invention

In a second embodiment of the microbicidal and antiseptic ointment, at least one kind selected from the group consisting of chlorhexidine and a salt thereof is present in an amount from about 0.1 to 4 mass % and a hydrocarbon alcohol having 2 to 3 carbon atoms is present in an amount from about 8 to 25 mass %.

Examples and proportions of chlorhexidine and a salt thereof, the same of a hydrocarbon alcohol, other ingredients, and reasons that the numerical values regarding those ingredients are limited, etc. are the same as described in the first embodiment of the microbicidal and antiseptic ointment according to the present invention.

When the second embodiment of the microbicidal and antiseptic ointment has the above-mentioned composition, it can exhibit a significant fast-acting and persistent microbicidal effect.

Method of preparing compositions

According to the present invention, the first and second embodiments of the microbicidal and antiseptic ointment can be prepared by incorporating the above-mentioned ingredients in reference to known methods, for example, methods described in general rules for preparations of the Japanese Pharmacopoeia, 14th Edition, JP-B No. 07-116020, JP-A Nos. 2003-212716, 2001-181180, 2004-307353, 11-76799, 11-76798 or 8-291049.

An emulsion ointment can be prepared as the following procedures. Chlorhexidine and/or a salt thereof, a hydrocarbon alcohol having 2 to 3 carbon atoms, a surfactant and, if necessary, any other ingredient are added to an aqueous base, followed by heating and mixing, and dissolved in the base. The resultant is mixed with an oily base by stirring.

A gel ointment can be prepared as the following procedures. Chlorhexidine and/or a salt thereof, a hydrocarbon alcohol having 2 to 3 carbon atoms and, if necessary, any other ingredient are added to an ointment base, followed by heating and mixing.

(III) Antiseptic method

The present invention discloses an antiseptic method, in which the first or second embodiment of the ointment described above herein is applied to human hands or skin.

Any antiseptic method can be adopted. Exemplary methods of disinfecting hands or skin include a rubbing method etc. In the rubbing method, the microbicidal and antiseptic ointment of the present invention is placed on a palm and rubbed into the skin to achieve the antisepsis. A dose of the microbicidal and antiseptic ointment herein is not particularly limited because it may range depending on the site in need of disinfection or a size thereof, but it is usually about 0.5 to 10 g. According to the present invention, since the microbicidal and antiseptic ointment can sustain the microbicidal effect for 6 hours or more, it is preferred that the microbicidal and antiseptic ointment herein is applied in a dose of about 0.5 to 10 g every 6 hours to maintain the effect for a longer time period than 6 hours.

(IV) Use

The use of the present invention is the use of the first or second embodiment of the ointment described above herein as a microbicidal and antiseptic ointment or the use thereof in the manufacture of a microbicidal and antiseptic ointment.

The present invention will, hereinafter, be described in more detail with examples and tests being referred, but it is not limited thereto.

Examples

Examples 1 to 3 and reference examples 1 and 2

The components in the oily phase in Table 1 were heated to 75 to 80°C, dissolved by stirring and then adjusted to a temperature of 60 $\pm$ 2°C. The components in the aqueous phase were heated to 65 to 70°C to dissolve surfactants thoroughly and then adjusted to a temperature of 60 $\pm$ 2°C. Next, the oily phase was gradually poured into the aqueous phase while being stirred with a homomixer. Following this, the resultant was further stirred at 8000 rpm for 2 minutes and cooled down to room temperature while being stirred, finally to obtain the ointments described in examples 1, 2 and 3 (preparations 1, 2 and 3, respectively) and described in reference examples 1 and 2 (reference preparations 1 and 2, respectively) in Table 1.

Table 1

| | | Amount of Ingredient (mass %) | | | | |
|---|---|---|---|---|---|---|
| | | Example 1 | Example 2 | Example 3 | Reference example 1 | Reference example 2 |
| Oily phase | Cetostearyl alcohol | 6.0 | 6.0 | 6.0 | 6.0 | 6.0 |
| | Palmitic acid | 0.5 | 0.5 | 0.5 | 0.5 | 0.5 |
| | Stearic acid | 0.5 | 0.5 | 0.5 | 0.5 | 0.5 |
| | Silicone oil | 2.0 | 2.0 | 2.0 | 2.0 | 2.0 |
| | Glyceryl monostearate (HLB 3) | 2.0 | 2.0 | 2.0 | 2.0 | 2.0 |
| | Petrolatum | 2.0 | 2.0 | 2.0 | 2.0 | 2.0 |
| | Squalane | 3.0 | 3.0 | 3.0 | 3.0 | 3.0 |
| | Liquid paraffin | 10.0 | 10.0 | 10.0 | 10.0 | 10.0 |

(continued)

| | | Amount of Ingredient (mass %) | | | | |
|---|---|---|---|---|---|---|
| | | Example 1 | Example 2 | Example 3 | Reference example 1 | Reference example 2 |
| Aqueous phase | Chlorhexidine gluconate | 0.2 | 0.2 | 0.1 | 0.5 | 0.5 |
| | Ethanol | 10.0 | 20.0 | 10.0 | 5.0 | - |
| | POE 60 mol hydrogenated castor oil(HLB 15) | 6.0 | 6.0 | 6.0 | 6.0 | 6.0 |
| | Sorbitol | 5.0 | 5.0 | 5.0 | 5.0 | 5.0 |
| | 1,3-butylene glycol | 10.0 | 10.0 | 10.0 | 10.0 | 10.0 |
| | Purified water | To make 100 | To make 100 | To make 100 | To make 100 | To make 100 |

Example 4

| | | |
|---|---|---|
| (1) Cetyl alcohol | | 4.0 mass % |
| (2) Stearic acid | | 1.5 mass % |
| (3) Palm oil triglyceride | | 1.0 mass % |
| (4) Glyceryl tri-2-ethylhexanoate | | 2.0 mass % |
| (5) Silicone oil | | 2.5 mass % |
| (6) Squalane | | 4.0 mass % |
| (7) POE (30) cholesteryl ether | | 4.0 mass % |
| (8) Glyceryl monostearate | | 1.0 mass % |
| (9) Ethanol | | 9.0 mass % |
| (10) Propylene glycol | | 18.0 mass % |
| (11) Glycerin | | 12.0 mass % |
| (12) Sodium pyrrolidone carboxylate | | 2.0 mass % |
| (13) Chlorhexidine gluconate | | 0.5 mass % |
| (14) Betaine | | 3.0 mass % |
| (15) Purified water | | q.s. |
| | Total | 100.0 mass % |

The components in the above-described (1) to (10) were heated to 75 to 80°C, dissolved and then adjusted to a temperature of 70 $\pm$ 2°C to prepare the oily phase. In the meanwhile, the components in the above-described (11) to (15) were heated to 70 to 75 °C and adjusted to a temperature of 70 $\pm$ 2°C to prepare the aqueous phase. Subsequently, the oily phase was gradually poured into the aqueous phase while being stirred with a homomixer. Following this, the resultant was further stirred at 8000 rpm for 2 minutes, cooled down to 45°C while being stirred and treated with a microfluidizer, finally to obtain a creamy ointment.

Test Example

The evaluation of the microbicidal effect of preparation 1 by a palm stamp method

1. Test preparations

(1) Preparation 1

(2) Preparation 2

(3) Reference preparation 1

(4) Reference preparation 2

(5) Reference preparation 3: 0.2 w/v% chlorhexidine alcohol lotion (WELLUP manufactured by Maruishi Pharmaceutical Co., Ltd., containing 0.2 g (0.2 w/v%) of chlorhexidine gluconate in the total volume of 100 ml)

(6) Ethanol for disinfection

2. Test method

According to the palm stamp method, microbial sampling was performed by pushing a whole palm against a hand-shaped medium (Hand Petan Check (trademark registered in Japan by Eiken Kizai Co., LTD.)). After the whole palm was pushed against the hand-shaped medium, the hand-shaped medium was incubated at 35°C for 18 hours. After incubation, colonies were counted and the count was referred to as microbial count in the palm. Average microbial count in the palm before disinfection was calculated and referred to as a baseline.

The microbial counts in the palm before (i.e., baseline), immediately after, 3 hours after and 6 hours after disinfection were measured, and then the reduction ratios of the microbial counts after disinfection to the baseline were calculated to evaluate the efficacy.

Disinfection was performed according to the following procedures. After washing both hands with soap and drying off the hands with a sterile towel, a subject applied to the hands 3 g of preparation 1 or 2, or reference preparation 1 or 2, and then rubbed the preparation into the both hands for 2 to 3 minutes. Disinfection with reference preparation 3 or ethanol for disinfection was performed according to the following procedures. After washing both hands with soap and drying off the hands with a sterile towel, a subject rubbed into the both hands 3 ml of reference preparation 3 or ethanol for disinfection for 3 times. After disinfection, the subject put surgical gloves on the both hands.

The microbial count in the palm before disinfection means the microbial count in the palm immediately after washing both hands with soap and drying the hands off with a sterile towel.

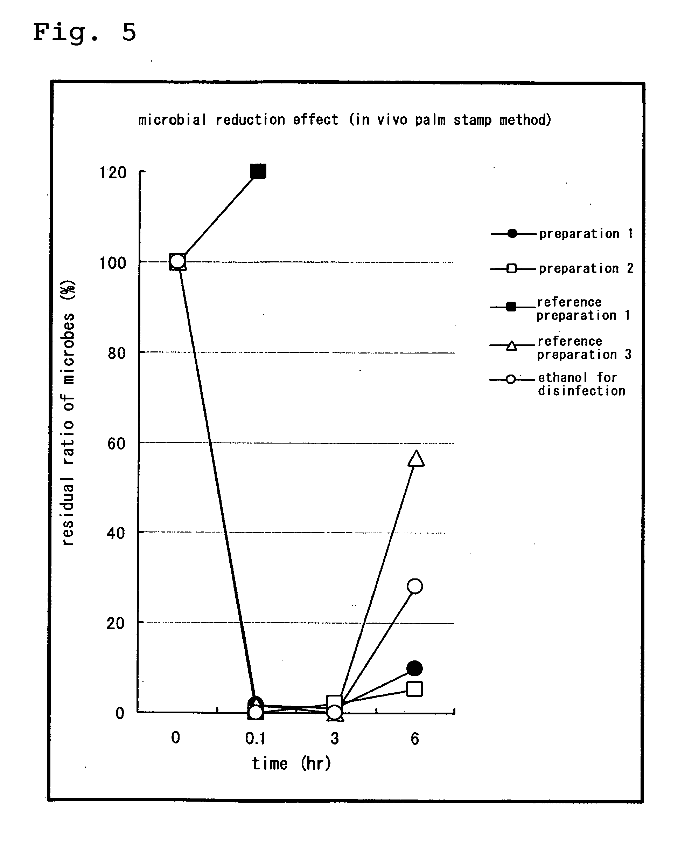

The microbial count immediately after disinfection means the microbial count in the palm before gloves were put on hands after the disinfection described above (the time is equivalent to 0.1 hour in Fig. 5).

The microbial count 3 hours after the disinfection means the microbial count in the right palm 3 hours after rubber gloves were put on hands.

The microbial count 6 hours after the disinfection means the microbial count in the left palm 6 hours after rubber gloves were put on hands.

3. Results

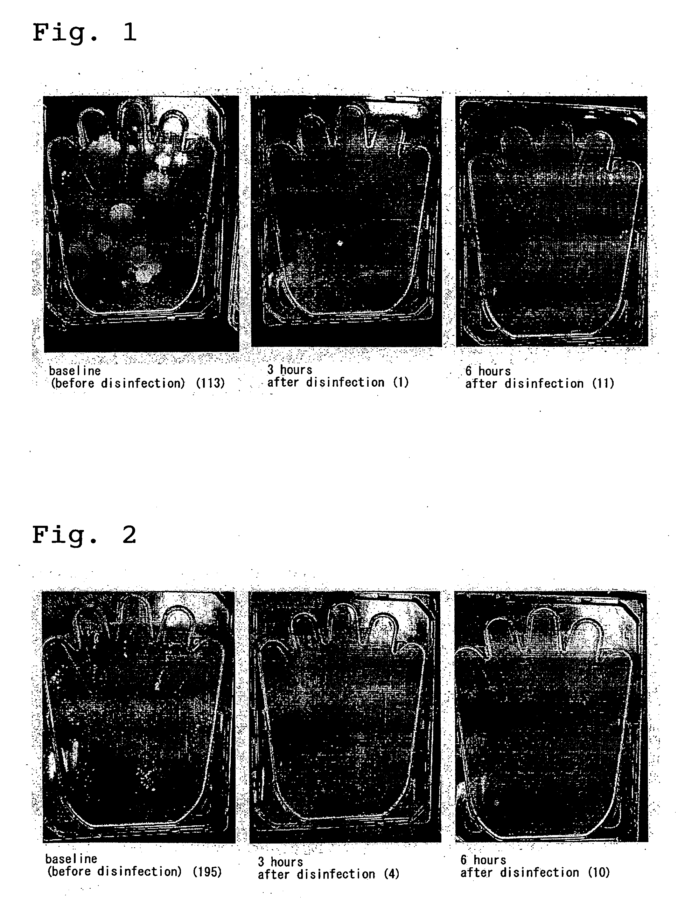

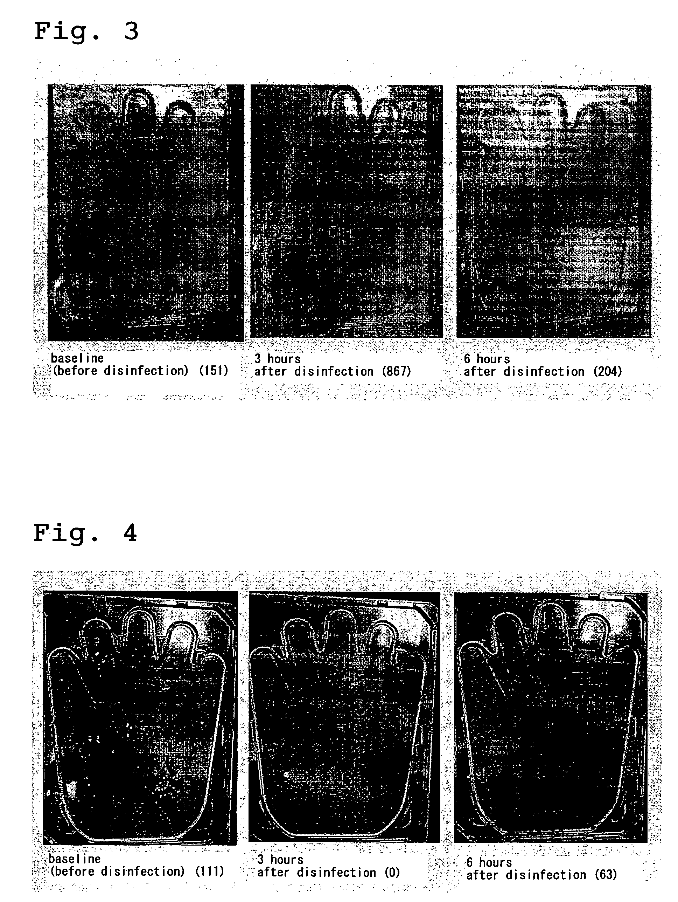

Figs. 1 and 2 show the results of preparations 1 and 2, respectively, while Figs. 3 and 4 show the results of reference preparations 1 and 3, respectively. In addition, Fig. 5 shows the microbial reduction effects of preparations 1 and 2, reference preparations 1 and 3, and ethanol for disinfection. As Figs. 1, 2 and 5 show, preparations 1 and 2 allowed no microbial growth even 6 hours after disinfection and manifested a prolonged microbicidal effect. In contrast, as Figs. 3 and 5 show, reference preparation 1 demonstrated no microbicidal and antiseptic effect. Additionally, reference preparation 2 demonstrated no antiseptic effect even immediately after disinfection. As Figs. 4 and 5 show, reference preparation 3 sustained the microbicidal effect until 3 hours after disinfection, but 6 hours after disinfection, it allowed the microbial reduction ratio to fall as evaluated by the palm stamp method and let microbial growth to be seen in the palm. As Fig. 5 shows, ethanol for disinfection sustained the microbicidal effect until 3 hours after disinfection, but 6 hours after disinfection, it allowed the antiseptic effect to fall as evaluated by the palm stamp method.

INDUSTRIAL APPLICABILITY

The microbicidal and antiseptic ointment of the present invention is useful for application on the skin of palms or other parts.

**Claims**

1. A microbicidal and antiseptic ointment, comprising at least one kind selected from the group consisting of chlorhexidine and a salt thereof in an amount from 0.1 to 4 mass % and a hydrocarbon alcohol having 2 to 3 carbon atoms in an amount from 8 to 25 mass %, wherein the ointment sustains a microbicidal effect for at least 6 hours, the microbicidal effect being approved by a test method for evaluation of hand antisepsis.

**2.** The microbicidal and antiseptic ointment of claim 1, wherein the test method for evaluation of hand antisepsis is a glove juice method, a palm stamp method or a finger streak plate method.

**3.** The microbicidal and antiseptic ointment of claim 1, wherein the ointment is an emulsion ointment.

**4.** The microbicidal and antiseptic ointment of claim 3, wherein the emulsion ointment comprises (a) at least one oily base selected from the group consisting of a lipid, higher alcohol and higher fatty acid compound, (b) a nonionic surfactant and (c) at least one aqueous base selected from the group consisting of a water-soluble polyhydric alcohol and water.

**5.** The microbicidal and antiseptic ointment of claim 4, wherein the higher fatty acid compound is a higher fatty acid, higher fatty acid salt, higher fatty acid ester or higher fatty acid amide.

**6.** A method for disinfecting a human hand or skin, comprising applying to the human hand or skin an ointment which comprises at least one kind selected from the group consisting of chlorhexidine and a salt thereof in an amount from 0.1 to 4 mass % and a hydrocarbon alcohol having 2 to 3 carbon atoms in an amount from 8 to 25 mass %, and sustains a microbicidal effect for at least 6 hours, the microbicidal effect being approved by a test method for evaluation of hand antisepsis.

**7.** A use of an ointment as a microbicidal and antiseptic ointment, the ointment comprising at least one kind selected from the group consisting of chlorhexidine and a salt thereof in an amount from 0.1 to 4 mass % and a hydrocarbon alcohol having 2 to 3 carbon atoms in an amount from 8 to 25 mass %, wherein the ointment sustains a microbicidal effect for at least 6 hours, the microbicidal effect being approved by a test method for evaluation of hand antisepsis.

**8.** A microbicidal and antiseptic ointment, comprising at least one kind selected from the group consisting of chlorhexidine and a salt thereof in an amount from 0.1 to 4 mass % and a hydrocarbon alcohol having 2 to 3 carbon atoms in an amount from 8 to 25 mass %.

**9.** A method for disinfecting a human hand or skin, comprising applying to the human hand or skin an ointment which comprises at least one kind selected from the group consisting of chlorhexidine and a salt thereof in an amount from 0.1 to 4 mass % and a hydrocarbon alcohol having 2 to 3 carbon atoms in an amount from 8 to 25 mass %.

**10.** A use of an ointment as a microbicidal and antiseptic ointment, the ointment comprising at least one kind selected from the group consisting of chlorhexidine and a salt thereof in an amount from 0.1 to 4 mass % and a hydrocarbon alcohol having 2 to 3 carbon atoms in an amount from 8 to 25 mass %.

# Fig. 1

baseline
(before disinfection) (113)

3 hours
after disinfection (1)

6 hours
after disinfection (11)

# Fig. 2

baseline
(before disinfection) (195)

3 hours
after disinfection (4)

6 hours
after disinfection (10)

Fig. 3

baseline
(before disinfection) (151)

3 hours
after disinfection (867)

6 hours
after disinfection (204)

Fig. 4

baseline
(before disinfection) (111)

3 hours
after disinfection (0)

6 hours
after disinfection (63)

# Fig. 5

EP 1 990 050 A1

<table>
<tr><td colspan="2">INTERNATIONAL SEARCH REPORT</td><td>International application No.<br>PCT/JP2007/052554</td></tr>
</table>

A.  CLASSIFICATION OF SUBJECT MATTER
*A61K31/155*(2006.01)i, *A61K9/06*(2006.01)i, *A61K47/10*(2006.01)i, *A61K47/12*
(2006.01)i, *A61K47/14*(2006.01)i, *A61K47/16*(2006.01)i

According to International Patent Classification (IPC) or to both national classification and IPC

B.   FIELDS SEARCHED

Minimum documentation searched (classification system followed by classification symbols)
A61K31/155, A61K9/06, A61K47/10, A61K47/12, A61K47/14, A61K47/16

Documentation searched other than minimum documentation to the extent that such documents are included in the fields searched
Jitsuyo Shinan Koho          1922-1996    Jitsuyo Shinan Toroku Koho   1996-2007
Kokai Jitsuyo Shinan Koho    1971-2007    Toroku Jitsuyo Shinan Koho   1994-2007

Electronic data base consulted during the international search (name of data base and, where practicable, search terms used)
CAplus(STN), REGISTRY(STN), JMEDPlus(JDream2), JST7580(JDream2), JSTPlus(JDream2)

C.   DOCUMENTS CONSIDERED TO BE RELEVANT

| Category* | Citation of document, with indication, where appropriate, of the relevant passages | Relevant to claim No. |
|---|---|---|
| X | JP 10-1443 A  (Sekisui Chemical Co., Ltd.),<br>06 January, 1998 (06.01.98),<br>Claim 1; examples 12 to 14; Par. Nos. [0009],<br>[0055]<br>(Family: none) | 1-5,8 |
| X | JP 2001-278764 A  (NOF Corp.),<br>10 October, 2001 (10.10.01),<br>Claims 1, 2; Par. No. [0004]<br>(Family: none) | 1-5,8 |
| X | JP 55-167217 A  (Nobuo MOCHIDA),<br>26 December, 1980 (26.12.80),<br>Full text;, particularly, examples 1, 2<br>(Family: none) | 1-5,8 |

☒  Further documents are listed in the continuation of Box C.       ☐  See patent family annex.

*     Special categories of cited documents:
"A"   document defining the general state of the art which is not considered   to be of particular relevance
"E"   earlier application or patent but published on or after the international filing date
"L"   document which may throw doubts on priority claim(s) or which is cited to establish the publication date of another citation or other special reason (as specified)
"O"   document referring to an oral disclosure, use, exhibition or other means
"P"   document published prior to the international filing date but later than the priority date claimed

"T"   later document published after the international filing date or priority date and not in conflict with the application but cited to understand the principle or theory underlying the invention
"X"   document of particular relevance; the claimed invention cannot be considered novel or cannot be considered to involve an inventive step when the document is taken alone
"Y"   document of particular relevance; the claimed invention cannot be considered to involve an inventive step when the document is combined with one or more other such documents, such combination being obvious to a person skilled in the art
"&"   document member of the same patent family

Date of the actual completion of the international search
22 March, 2007 (22.03.07)

Date of mailing of the international search report
03 April, 2007 (03.04.07)

Name and mailing address of the ISA/
Japanese Patent Office

Authorized officer

Facsimile No.

Telephone No.

Form PCT/ISA/210 (second sheet) (April 2005)

18

**INTERNATIONAL SEARCH REPORT**

International application No.

PCT/JP2007/052554

C (Continuation). DOCUMENTS CONSIDERED TO BE RELEVANT

| Category* | Citation of document, with indication, where appropriate, of the relevant passages | Relevant to claim No. |
|---|---|---|
| X | JP 10-17464 A (Lion Corp.), 20 January, 1998 (20.01.98), Example 3; Par. No. [0015] (Family: none) | 1-5,8 |
| X | JP 2002-193706 A (Kenei Pharmaceutical Co., Ltd.), 10 July, 2002 (10.07.02), Claim 1; Par. Nos. [0007], [0017] (Family: none) | 1-5,8 |
| Y | JP 6-199700 A (Toko Yakuhin Kogyo Kabushiki Kaisha), 19 July, 1994 (19.07.94), Example 13, 14 & US 5750579 A & EP 604848 A2 | 1-5,8 |
| Y | JP 2001-220303 A (Tiporu Kabushiki Kaisha), 14 August, 2001 (14.08.01), Examples; table 4, 5; Par. No. [0065] (Family: none) | 1-5,8 |
| Y | WO 2004/093859 A1 (Sumitomo Pharmaceuticals Co., Ltd.), 04 November, 2004 (04.11.04), Full text;, particularly, example 5; test example 2 & EP 1618877 A1 & US 2007/025948 A1 | 1-5,8 |
| A | JP 11-279084 A (Lion Corp.), 12 October, 1999 (12.10.99), (Family: none) | 1-5,8 |
| A | JP 2000-273004 A (Nicca Chemical Co., Ltd.), 03 October, 2000 (03.10.00), (Family: none) | 1-5,8 |

Form PCT/ISA/210 (continuation of second sheet) (April 2005)

**INTERNATIONAL SEARCH REPORT**

International application No.

PCT/JP2007/052554

| Box No. II | Observations where certain claims were found unsearchable (Continuation of item 2 of first sheet) |
|---|---|

This international search report has not been established in respect of certain claims under Article 17(2)(a) for the following reasons:

1. ☒ Claims Nos.: 6, 7, 9, 10
   because they relate to subject matter not required to be searched by this Authority, namely:
   The inventions as set forth in claims 6, 7, 9 and 10 pertain to methods for treatment of the human body by therapy and thus relate to a subject matter which this International Searching Authority is not required to search (PCT Article 17(2)(a)(i) and PCT rule 39.1(iv)).

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

| Box No. III | Observations where unity of invention is lacking (Continuation of item 3 of first sheet) |
|---|---|

This International Searching Authority found multiple inventions in this international application, as follows:

1. ☐ As all required additional search fees were timely paid by the applicant, this international search report covers all searchable claims.

2. ☐ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**
the

☐ The additional search fees were accompanied by the applicant's protest and, where applicable, payment of a protest fee..

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (April 2005)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 3515821 B
- JP 3592080 B
- JP 2002179919 A
- JP 7116020 B
- JP 2003212716 A
- JP 2001181180 A
- JP 2004307353 A
- JP 11076799 A
- JP 11076798 A
- JP 8291049 A

**Non-patent literature cited in the description**

- Manual of the Japanese Pharmacopoeia. Hirokawa-shoten, 2001, 1225-1229
- *Proposed Rules: Federal Register,* 1994, vol. 59, 31401-52
- *Morbidity and Mortality Weekly Report,* 2002, vol. 51 (RR-16
- *Geka Shinryo,* April 1982, 513-518
- *Federal Register,* 1974, vol. 39 (179), 33137
- *Federal Register,* 1978, vol. 43 (4), 1242
- *Federal Register,* 1994, vol. 59 (116), 31444